# EUROPEAN PATENT APPLICATION

(11) **EP 2 020 448 A1**
(43) Date of publication of application: **04.02.2009**
(21) Application number: 07738578.9
(22) Date of filing: 14.03.2007
(51) Int. Cl.: C12Q 1/68, C12N 15/09, C12Q 1/04

(54) **PRIMER SET FOR DETECTION OF DEKKERA YEAST OR BRETTANOMYCES YEAST**

(30) Priority: 16.05.2006 JP 2006136810
(71) Applicant: KIRIN BEER KABUSHIKI KAISHA, Tokyo 104-8288 (JP)
(72) Inventor: HAYASHI, Nobuyuki, Tokyo 104-8288 (JP); ARAI, Ritsuko, Tokyo 104-8288 (JP); KOMODA, Toshikazu, Tokyo 104-8288 (JP)
(74) Representative: HOFFMANN EITLE
(86) International application number: PCT/JP2007/055108
(87) International publication number: WO 2007/132589

(57) **Abstract**

An object of the present invention is to provide a primer set for use in a LAMP method, which can accurately, rapidly and simply identify yeast species of genus Dekkera and yeast species of genus Brettanomyces. According to the present invention, there is provided a LAMP primer set for use in the detection of the yeast species of genus Dekkera and the yeast species of genus Brettanomyces, which comprises primers selected from the group consisting of the polynucleotides having the base sequences of SEQ ID NOS: 1 to 21 or the homologous polynucleotides thereof.

## Description

### Technical Field

The present invention relates to a primer set for use in the detection of a yeast of genus Dekkera and a yeast of genus Brettanomyrces, which are relevant to a decrease in the quality of alcoholic beverages or soft drinks. Further, the present invention relates to a method for detecting and quantifying the yeast of genus Dekkera and the yeast of genus Brettanomyces, using this primer set.

### Background Art

Deterioration of beverages due to contamination by yeast and proliferation of the yeast therein causes economical damage. Such quality accident takes place over the world. It has been known that a yeast of genus Dekkera (asexual generation of which is referred to as genus Brettanomyces), which is considered to be a yeast causing such deterioration, causes turbidity and unpleasant odor in beer, wine and soft drinks (Kurtzman, C. P. & Fell, J. W. The Yeasts, A taxonomic Study, 4th edition, 1998, Elsevier Science B. V., The Netheriands).

In beer manufacturing industry, the yeast of genus Dekkera generates acetic acid or acetates when it proliferates in ordinary beer, so that it causes damage to the quality of beer (Back, W.: Farbatlas und Handbuch der Geraenkebiologie, Teil I, 1994, Verlag Hans Carl, Nuernberg, European Brewery Convention: ANALYTICA-MICROBIOLOGICA-EBC, 2nd ed. 2005 Fachverlag Hans Carl, Nuernberg). In wine manufacturing industry, the yeast of genus Dekkera generates phenol odor substances such as 4-ethylphenol, amines, acetic acid, etc.
The odor of wine contaminated by such a yeast is expressed with terms such as "animal-like" or "barnyard-like," and thus the commercial value of such wine is reduced (Mitrakul et al.: Food Microbiology, 1999, Vol. 16, 3-14, Phister et al.: Applied and Environmental Microbiology, 2003, Vol. 69, 7430-7434, Silva et al.: Am. J. Enol. Vitic., 2004, Vol. 55, 65-72).

In soft drink manufacturing industry, the yeast of genus Dekkera generates a large amount of acid such as acetic acid from glucose or other carbon sources, and such acid brings on unique odor caused by acetate or the like (Back, W.: Farbatias und Handbuch der Geraenkebiologie, Teil II, 1999, Verlag Hans Carl, Nuernberg).
Thus, when the yeast of genus Dekkera proliferates in a product or during a production process, it has a great influence on the industry. Accordingly, a technique of rapidly detecting and/or identifying such a yeast is important for the control of quality.

A traditional method for detecting a wild-type yeast is a culture method. In the case of culturing the yeast of genus Dekkera, a selective medium, to which cycloheximide has been added, is used, and such culture generally requires approximately 1 to 2 weeks. Moreover, a method for biochemically identifying this yeast requires 3 to 4 weeks, and the results are often unclear (Phister et al: Applied and Environmental Microbiology, 2003, Vol. 69, 7430-7434, Silva et al.: Am. J. Enol. Vitic., 2004, Vol. 55, 65-72).

Several methods for detecting the yeast of genus Dekkera in a molecular biological manner have been reported so far. Ibeas et al. (Applied and Environmental Microbiology, 1996, Vol. 62, 998-1003) have reported that approximately 10 cells of the yeast of genus Dekkera could be detected in a contaminated sherry wine by two-step PCR. Hyldig-Nielsen et al. (WO00/77259) and Stender et al. (Applied and Environmental Microbiology, 2001, Vol. 67, 938-941) have developed a peptide nucleic acid probe used for the 26S rRNA gene of Dekkera bruxeliensis, and they have developed a method for detecting and identifying such Dekkera bruxellensis from wine according to a fluorescence in situ hybridization method. Delaherche et al. (Journal of Applied Microbiology, 2004, Vol. 97, 910-915) and Phister et al. (Applied and Environmental Microbiology, 2003, Vol. 69, 7430-7434) have reported a technique of detecting Dekkera bruxellensis from wine by a real-time PCR technique.

Moreover, with regard to the identification of the yeast of genus Dekkera at a species level, there have been the following reports. Specifically, Mitrakul et al. (Food Microbiology, 1999, Vol. 16, 3-14) have reported that yeasts of genus Dekkera could be distinguished at a species or strain level by RAPD PCR. Furthermore, Cocolin et al. (FEMS Microbiol. Lett., 2000, Vol. 189, 81-87) have reported a method comprising directly amplifying the genes of the yeast of genus Dekkera contained in wine according to PCR and then distinguishing them by denaturing gradient gel electrophoresis (DGGE).

Egli et al. (Am. J. Enol. Vitic., 2001, Vol. 52, 241-247) have amplified by PCR two internal transcribed spacer (ITS) regions sandwiched by ribosome RNA genes of the yeast of genus Dekkera and a 5.8S rRNA gene existing between the two regions, and they have then distinguished them based on the diversity of patterns of fragments obtained by a restriction enzyme treatment.

For the studies of the flora of the yeast of genus Dekkera in the brewing of a traditional Irish cider, Morrissey et al. (Journal of Applied Microbiology, 2004, Vol. 97, 647-655) have analyzed the PCR amplification products of the ITS regions and the 5.8S rRNA gene by a restriction enzyme cleavage analysis.

However, since the PCR method, the real-time PCR method, and the fluorescence in situ hybridization method require high-level temperature control and fluorescence observation, these methods need expensive apparatuses. In addition, the two-step PCR method, the RAPD PCR method, the treatment of an amplified product with restriction enzymes, and the DGGE method require a long period of time and complicated operations. Thus, these methods have been problematic when they have been carried out in daily microorganism tests.

A nucleic acid amplification technique known as a LAMP (loop mediated isothermal amplification) method is characterized in that the reaction progresses at an equal temperature, in that the method has high specificity because it uses 4 types of primers that recognize 6 regions, in that the method has high amplification efficiency and thus it enables amplification in a short time, and in that the method provides a large amount of amplification product and it is suitable for simple detection (WO00/28082 and T. Notomi et al.: Nucleic Acids research, 2000, 28(12), e63). Tsuchiya et al. have developed a primer(s) for use in a LAMP method for detecting the yeasts of genus Brettanomyces and genus Dekkera from the D2 regions of the rRNA genes of the yeasts of these genera (WO2005/093059).

However, a primer set for used in a LAMP method, which targets for the ITS regions of the yeast of genus Brettanomyces and the yeast of genus Dekkera, has been unknown so far.

### Summary of the Invention

The present inventors have succeeded in producing a primer set for use in a LAMP method, which can accurately identify the yeast of genus Dekkera and the yeast of genus Brettanomyces and which can also quantify them.

It is an object of the present invention to provide a primer set for use in a LAMP method, which can accurately, rapidly and simply identify the yeast of genus Dekkera and the yeast of genus Brettanomyces at a species level and which can also quantify them accurately.

Hereinafter, an invention relating to the detection of Dekkera anomala is defined as a first embodiment, an invention relating to the detection of Dekkera bruxellensis is defined as a second embodiment, an invention relating to the detection of Dekkera custersiana is defined as a third embodiment, and an invention relating to the detection of Brettanomyces naardenensis is defined as a fourth embodiment.

### First embodiment

According to the first embodiment of the present invention, there is provided a LAMP primer set for use in the detection of Dekkera anomala (hereinafter referred to as the "primer set of the first embodiment"), which comprises the following polynucleotides:
a polynucleotide (FIP) having the base sequence of SEQ ID NO: 1, or a polynucleotide consisting of at least 10 bases, which hybridizes with a polynucleotide having a sequence complementary to the base sequence (polynucleotide 1);
a polynucleotide (F3) having the base sequence of SEQ ID NO: 2, or a polynucleotide consisting of at least 10 bases, which hybridizes with a polynucleotide having a sequence complementary to the base sequence (polynucleotide 2);
a polynucleotide (BIP) having the base sequence of SEQ ID NO: 3, or a polynucleotide consisting of at least 10 bases, which hybridizes with a polynucleotide having a sequence complementary to the base sequence (polynucleotide 3); and
a polynucleotide (B3) having the base sequence of SEQ ID NO: 4, or a polynucleotide consisting of at least 10 bases, which hybridizes with a polynucleotide having a sequence complementary to the base sequence (polynucleotide 4).

According to the first embodiment of the present invention, there is also provided a method for detecting Dekkera anomala, which comprises performing a nucleic acid amplification reaction by a LAMP method using a primer set consisting of polynucleotides 1, 2, 3 and 4.

### Second embodiment

According to the second embodiment of the present invention, there is provided a LAMP primer set for use in the detection of Dekkera bruxellensis (hereinafter also referred to as the "primer set of the second embodiment"), which comprises the following polynucleotides:
a polynucleotide (FIP) having the base sequence of SEQ ID NO: 7, or a polynucleotide consisting of at least 10 bases, which hybridizes with a polynucleotide having a sequence complementary to the base sequence (polynucleotide 7);
a polynucleotide (F3) having the base sequence of SEQ ID NO: 8, or a polynucleotide consisting of at least 10 bases, which hybridizes with a polynucleotide having a sequence complementary to the base sequence (polynucleotide 8);
a polynucleotide (BIP) having the base sequence of SEQ ID NO: 9, or a polynucleotide consisting of at least 10 bases, which hybridizes with a polynucleotide having a sequence complementary to the base sequence (polynucleotide 9); and
a polynucleotide (B3) having the base sequence of SEQ ID NO: 10, or a polynucleotide consisting of at least 10 bases, which hybridizes with a polynucleotide having a sequence complementary to the base sequence (polynucleotide 10).

According to the second embodiment of the present invention, there is also provided a method for detecting Dekkera bruxellensis, which comprises performing a nucleic acid amplification reaction by a LAMP method using a primer set consisting of polynucleotides 7, 8, 9 and 10.

### Third embodiment

According to the third embodiment of the present invention, there is provided a LAMP primer set for use in the detection of Dekkera custersiana (hereinafter referred to as the "primer set of the third embodiment"), which comprises the following polynucleotides:
a polynucleotide (FIP) having the base sequence of SEQ ID NO: 12, or a polynucleotide consisting of at least 10 bases, which hybridizes with a polynucleotide having a sequence complementary to the base sequence (polynucleotide 12);
a polynucleotide (F3) having the base sequence of SEQ ID NO: 13, or a polynucleotide consisting of at least 10 bases, which hybridizes with a polynucleotide having a sequence complementary to the base sequence (polynucleotide 13);
a polynucleotide (BIP) having the base sequence of SEQ ID NO: 14, or a polynucleotide consisting of at least 10 bases, which hybridizes with a polynucleotide having a sequence complementary to the base sequence (polynucleotide 14); and
a polynucleotide (B3) having the base sequence of SEQ ID NO: 15, or a polynucleotide consisting of at least 10 bases, which hybridizes with a polynucleotide having a sequence complementary to the base sequence (polynucleotide 15).

According to the third embodiment of the present invention, there is also provided a method for detecting Dekkera custersiana, which comprises performing a nucleic acid amplification reaction by a LAMP method using a primer set consisting of polynucleotides 12, 13, 14 and 15.

### Fourth embodiment

According to the fourth embodiment of the present invention, there is provided a LAMP primer set for use in the detection of Brettanomyces naardenensis (hereinafter referred to as the "primer set of the fourth embodiment"), which comprises the following polynucleotides:
a polynucleotide (FIP) having the base sequence of SEQ ID NO: 17, or a polynucleotide consisting of at least 10 bases, which hybridizes with a polynucleotide having a sequence complementary to the base sequence (polynucleotide 17);
a polynucleotide (F3) having the base sequence of SEQ ID NO: 18, or a polynucleotide consisting of at least 10 bases, which hybridizes with a polynucleotide having a sequence complementary to the base sequence (polynucleotide 18);
a polynucleotide (BIP) having the base sequence of SEQ ID NO: 19, or a polynucleotide consisting of at least 10 bases, which hybridizes with a polynucleotide having a sequence complementary to the base sequence (polynucleotide 19); and
a polynucleotide (B3) having the base sequence of SEQ ID NO: 20, or a polynucleotide consisting of at least 10 bases, which hybridizes with a polynucleotide having a sequence complementary to the base sequence (polynucleotide 20).

According to the fourth embodiment of the present invention, there is also provided a method for detecting Brettanomyces naardenensis, which comprises performing a nucleic acid amplification reaction by a LAMP method using a primer set consisting of polynucleotides 17, 18, 19 and 20.

According to the primer sets according to the present invention, the yeast of genus Dekkera and the yeast of genus Brettanomyces can be accurately identified at a species level with extremely high detection sensitivity, and also, the yeast of genus Dekkera and the yeast of genus Brettanomyces can be detected with high sensitivity. Further, the primer sets according to the present invention can be used in a nucleic acid amplification reaction by a LAMP method, and in a detection of a target species based on the presence or absence of an amplified product. Thus, according to the primer sets of the present invention, the yeast of genus Dekkera and the yeast of genus Brettanomyces can be accurately, rapidly and simply indentified at a species level.

According to the primer sets according to the present invention, the number of cells of the yeast of genus Dekkera and the yeast of genus Brettanomyces contained in a sample can be measured. Thus, According to the primer sets according to the present invention, the yeast of genus Dekkera and the yeast of genus Brettanomyces can be accurately quantified.

The yeast of genus Dekkera and the yeast of genus Brettanomyces are yeast species that cloud various types of beverages such as alcoholic beverages and soft drinks. Thus, the presence or absence of these yeast species may be used as an indicator of the quality control of various types of beverages. Accordingly, the primer sets according to the present invention is useful for the quality control of various types of beverages (for example, alcoholic beverages and soft drinks, and particularly, beer, low-malt beer (happoshu), and wine) and the examination of environmental samples.

### Brief Description of the Drawings

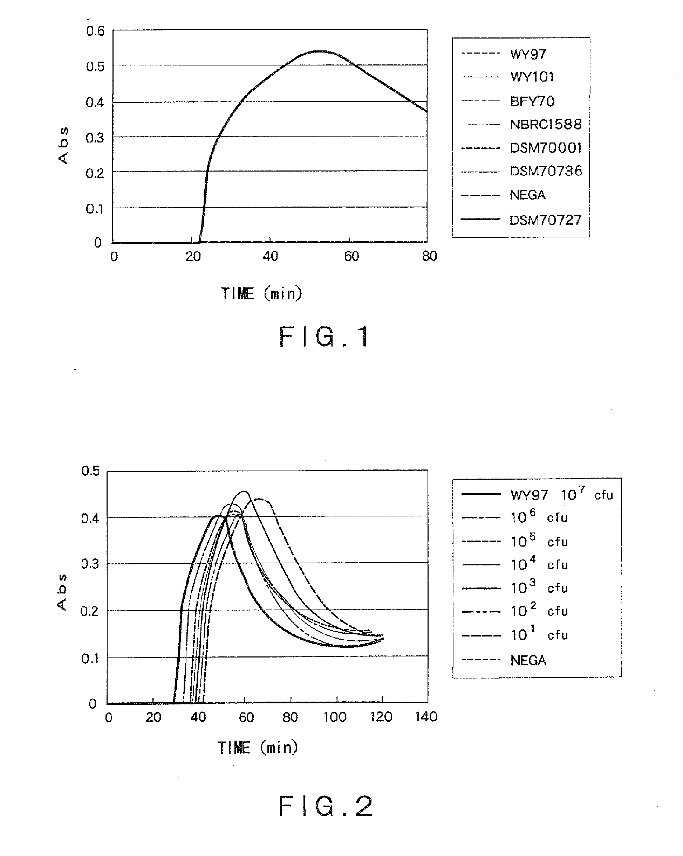

Figure 1 shows the reaction specificity of a primer set (DA1LF1LB1) for use in the detection of Dekkera anomala for detection target species. The following strains were used. WY97: Dekkera bruxellensis separated at a brewery; WY101: Saccharomyces cerevisiae separated at a brewery; BFY70: a yeast for use in brewing; NBRC1588: Brettanomyces naardenensis; DSM70001: Dekkera bruxellensis; DSM70736: Dekkera custersiana; Nega: no genomic DNA added; DSM70727: Dekkera anomala.
Figure 2 shows the detection results of a LAMP method obtained in the case of using a stepwisely diluted DNA solution as a detection sample (WY97). DB1LF1 was used as a LAMP primer set.

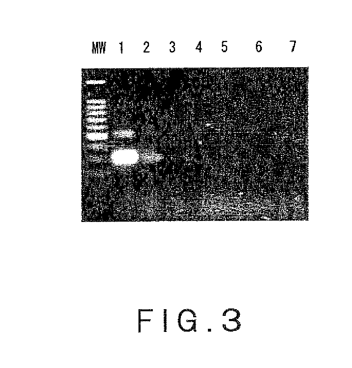

Figure 3 shows the detection results of a PCR method obtained in the case of using a stepwisely diluted DNA solution as a detection sample (WY97). The DNA sequences of SEQ ID NOS: 22 and 23 were used as a primer set in the PCR method. MW: a molecular weight marker; lane 1: 10⁷ cfu (WY97); lane 2: 10⁶ cfu; lane 3: 10⁵ cfu; lane 4: 10⁴ cfu; lane 5: 10³ cfu; lane 6: 10² cfu; lane 7: 10¹ cfu.

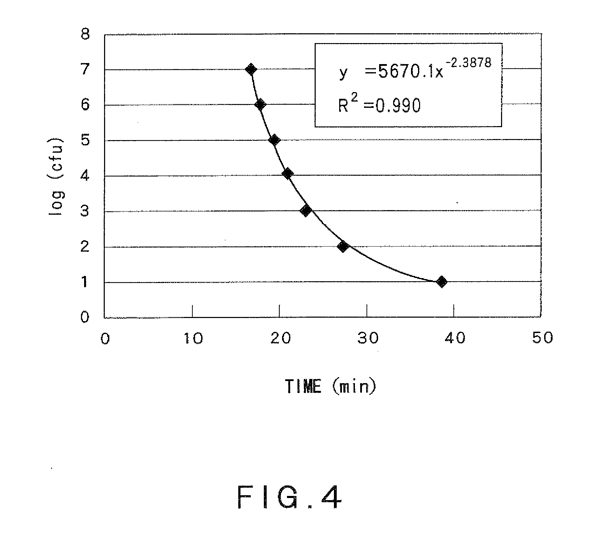

Figure 4 shows an approximate curve formed by the colony formation number of Dekkera anomala (ATCC56868) and the detection time by the LAMP method using DA1LF1LB1. The threshold time on the horizontal axis indicates the reaction time when the turbidity exceeded 0.1.

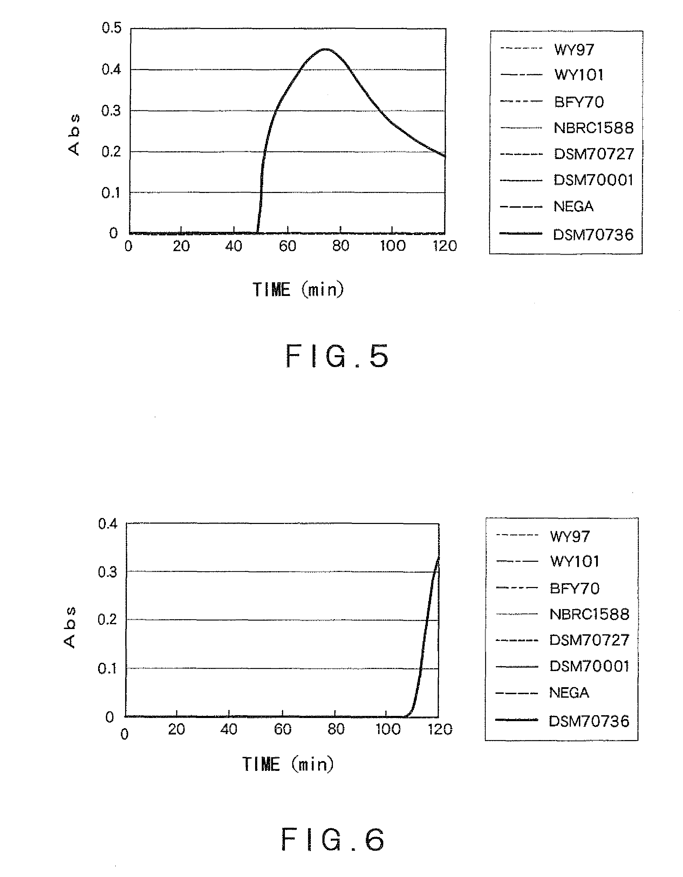

Figure 5 shows the detection reactivity of a primer set (DC2) for use in Dekkera custersiana. The following strains were used. WY97: Dekkera bruxellensis separated at a brewery; WY101: Saccharomyces cerevisiae separated at a brewery; BFY70: yeast for use in brewing; NBRC1588: Brettanomyces naardenensis; DSM70727: Dekkera anomala; DSM70001: Dekkera bruxellensis; Nega: no genomic DNA added; DSM70736: Dekkera custersiana.
Figure 6 shows the detection reactivity of a control primer set (DC1) used as a control of the primer set for use in the detection of Dekkera custersiana. The following strains were used. WY97: Dekkera bruxellensis separated at a brewery; WY101: Saccharomyces cerevisiae separated at a brewery; BFY70: yeast for use in brewing; NBRC1588: Brettanomyces naardenensis; DSM70727: Dekkera anomala; DSM70001: Dekkera bruxellensis; Nega: no genomic DNA added; DSM70736: Dekkera custersiana.

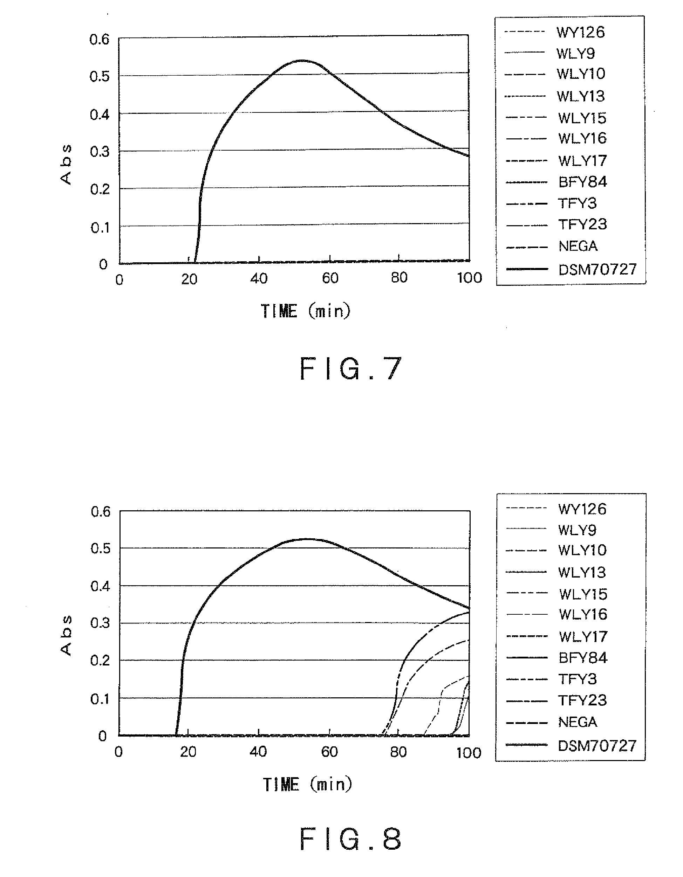

Figure 7 shows the detection reactivity of a primer set (DA1LF1LB1) for use in Dekkera anomala. The following strains were used. WY126: a wild-type yeast separated at a brewery; WLY9.WLY10.WLY13.WLY15.WLY16.WLY17: wild-type yeast separated from wine; BFY84-TFY3-TFY23: yeasts for use in brewing; Nega: no genomic DNA added; DSM70727: Dekkera anomala.
Figure 8 shows the detection reactivity of a control primer set (DA2LF1LB1) used as a control of the primer set for use in the detection of Dekkera anomala. The following strains were used. WY126: a wild-type yeast separated at a brewery; WLY9·WLY10·WLY13·WLY15·WLY16·WLY17: wild-type yeast separated from wine; BFY84·TFY3TFY23: yeasts for use in brewing; Nega: no genomic DNA added; DSM70727: Dekkera anomala.

### Detailed Description of the Invention

### Primers and primer sets

The primer set according to the present invention consists of 4 types of primers, namely, FIP, F3, BIP and B3. These primers correspond to 6 regions on a target nucleotide sequence. Specifically, regions F3c, F2c, F1c, B1, B2 and B3 are determined in this order from the 3-terminal side to the 5'-terminal side on the target base sequence. Thereafter, 4 types of primers, namely, FIP, F3, BIP and B3 are produced with respect to the 6 regions. Herein, regions complementary to the regions F3c, F2c and F1c are F3, F2 and F1, respectively. In addition, regions complementary to the regions B1, B2 and B3 are B1c, B2c and B3c, respectively.

FIP is a primer produced in such a way that it has an F2 region complementary to the F2c region of the target sequence on the 3'-terminal side and that it has the same sequence as the F1c region of the target gene on the 5'-terminal side. If necessary, a restriction enzyme site may be introduced into the portion between F1c and F2 of the FIP primer.

F3 is a primer produced in such a way that it has an F3 region complementary to the F3c region of the target gene.

BIP is a primer produced in such a way that it has a B2 region complementary to the B2c region of the target sequence on the 3'-terminal side and that it has the same sequence as the B1c region of the target gene on the 5'-terminal side. If necessary, a restriction enzyme site may be introduced into the portion between B1c and B2 of the BIP primer.

B3 is a primer produced in such a way that it has a B3 region complementary to the B3c region of the target gene.

When restriction enzyme sites are contained in the FIP and BIP primers, an amplified product is treated with restriction enzymes after completion of the nucleic acid amplification reaction by the LAMP method, so that it can be observed that a single band is formed after performing electrophoresis. In this case, if the target sequence contains a restriction enzyme site, it may not be necessary to artificially introduce such a restriction enzyme site into the primers.

When the primer set according to the present invention is used, one or two types of Loop primers (an LF primer or an LB primer) may be added in order to accelerate the nucleic acid amplification reaction. Such a Loop primer is designed such that it is annealed to a region between F1 and F2 or a region between B1 and B2, and it is then added to the LAMP reaction system. Thus, these primers bind to loop portions that are not used in the nucleic acid amplification process, so that a nucleic acid reaction can be promoted using all the loop portions as origins, and so that the nucleic acid amplification reaction can be thereby accelerated (e.g. Japanese Patent Laid-Open Publication No. 2002-345499).

Specifically, the primer set of the first embodiment may further comprise, as a loop primer(s), any one or both of the following polynucleotides: a polynucleotide (LF) having the base sequence of SEQ ID NO: 5, or a polynucleotide consisting of at least 10 bases, which hybridizes with a polynucleotide having a sequence complementary to the base sequence (polynucleotide 5); and a polynucleotide (LB) having the base sequence of SEQ ID NO: 6, or a polynucleotide consisting of at least 10 bases, which hybridizes with a polynucleotide having a sequence complementary to the base sequence (polynucleotide 6).

The primer set of the second embodiment may further comprise, as a loop primer, a polynucleotide (LF) having the base sequence of (SEQ ID NO: 11, or a polynucleotide consisting of at least 10 bases, which hybridizes with a polynucleotide having a sequence complementary to the base sequence (polynucleotide 11).

The primer set of the third embodiment may further comprise, as a loop primer, a polynucleotide (LB) having the base sequence of SEQ ID NO: 16, or a polynucleotide consisting of at least 10 bases, which hybridizes with a polynucleotide having a sequence complementary to the base sequence (polynucleotide 16).

The primer set of the fourth embodiment may further comprise, as a loop primer, a polynucleotide (LF) having the base sequence of SEQ ID NO: 21, or a polynucleotide consisting of at least 10 bases, which hybridizes with a polynucleotide having a sequence complementary to the base sequence (polynucleotides 21).

In the present invention, not only the polynucleotides having the base sequences of SEQ ID NOS: 1 to 21, but also polynucleotides hybridizing with polynucleotides having sequences complementary to the base sequences of (SEQ ID NOS: 1 to 21 (hereinafter also referred to as "homologous polynucleotides") can be used as LAMP primers.

The term "hybridize" is used in the present specification to mean that a certain polynucleotide hybridizes with a target polynucleotide, but that it does not substantially hybridize with polynucleotides other than the target polynucleotide. Such hybridization can be carried out under stringent conditions. Herein, "stringent conditions" can be determined depending on the Tm(°C) of a double strand of a primer sequence and a complementary strand thereof, a necessary salt concentration, etc. A technique of selecting a sequence used as a probe and then determining stringent conditions suitable therefor is well known to persons skilled in the art. (Refer to e.g. J. Sambrook, E. F. Frisch, T. Maniatis; Molecular Cloning 2nd edition, Cold Spring Harbor Laboratory (1989), etc.) As such stringent conditions, a hybridization reaction is carried out at a temperature slightly lower than the Tm determined based on a nucleotide sequence (for example, a temperature that is approximately 0°C to 5°C lower than the Tm), in a suitable buffer solution commonly used in hybridization. In addition, as other stringent conditions, washing after the hybridization reaction is carried out in a high concentration of low-salt-concentration solution. Examples of such stringent conditions include washing conditions wherein washing is carried out in a 6 x SSC/0.05% sodium pyrophosphate solution at temperatures of 37°C (for an oligonucleotide consisting of approximately 14 bases), 48°C (for an oligonucleotide consisting of approximately 17 bases), 55°C (for an oligonucleotide consisting of approximately 20 bases) and 60°C (for an oligonucleotide consisting of approximately 23 bases).

The nucleotide length of a homologous polynucleotide is at least 10 bases.

The nucleotide length of each of the homologous polynucleotides of FIP and BIP may be preferably at least 30 bases (for example 30 to 60 bases), and more preferably at least 39 bases (for example, 39 to 52 bases). The nucleotide length of the homologous polynucleotide of FIP may be particularly preferably at least 39 bases (for example, 39 to 46 bases). The nucleotide length of the homologous polynucleotide of BIP may be particularly preferably at least 46 bases (for example, 46 to 52 bases).

Moreover, the nucleotide length of each of the homologous polynucleotides of F3, B3, LF and LB may be preferably at least 12 bases (for example 12 to 30 bases), and more preferably at least 16 bases (for example, 16 to 25 bases). The nucleotide length of the homologous polynucleotide of F3 may be particularly preferably at least 16 bases (for example, 16 to 20 bases and 16 to 25 bases). The nucleotide length of the homologous polynucleotide of B3 may be particularly preferably at least 19 bases (for example, 19 to 22 bases and 19 to 25 bases). The nucleotide length of each of the homologous polynucleotides of LF and LB may be particularly preferably at least 19 bases (for example, 19 to 22 bases and 19 to 25 bases).

Such a homologous polynucleotide may be a polynucleotide comprising at least 10, preferably at least 15, more preferably at least 18, and particularly preferably at least 20 contiguous nucleotides of the corresponding base sequence.

Examples of polynucleotides homologous to the polynucleotides having the base sequences of SEQ ID NOS: 1 to 21 are as follows.
- A homologous polynucleotide of FIP having the base sequence of SEQ ID NO: 1: a polynucleotide comprising at least 34 (34 to 42), more preferably at least 39 (39 to 42) contiguous nucleotides of SEQ ID NO: 1 (in which one or several mutations may be introduced) (wherein the nucleotide length may be at most 60 bases, preferably at most 52 bases, and more preferably at most 46 bases)
- A homologous polynucleotide of F3 having the base sequence of SEQ ID NO: 2: a polynucleotide comprising at least 16 (16 to 20), more preferably 18 (18 to 20) contiguous nucleotides of SEQ ID NO: 2 (in which one or several mutations may be introduced) (wherein the nucleotide length may be at most 30 bases, preferably at most 25 bases, and more preferably at most 22 bases)
- A homologous polynucleotide of BIP having the base sequence of SEQ ID NO: 3: a polynucleotide comprising at least 42 (42 to 51), more preferably at least 46 (46 to 51) contiguous nucleotides of SEQ ID NO: 3 (in which one or several mutations may be introduced) (wherein the nucleotide length may be at most 60 bases and preferably at most 52 bases)
- A homologous polynucleotide of B3 having the base sequence of SEQ ID NO: 4: a polynucleotide comprising at least 16 (16 to 21), more preferably 19 (19 to 21) contiguous nucleotides of SEQ ID NO: 4 (in which one or several mutations may be introduced) (wherein the nucleotide length may be at most 30 bases, preferably at most 25 bases, and more preferably at most 22 bases)
- A homologous polynucleotide of LF having the base sequence of SEQ ID NO: 5: a polynucleotide comprising at least 16 (16 to 22), more preferably 19 (19 to 22) contiguous nucleotides of SEQ ID NO: 5 (in which one or several mutations may be introduced) (wherein the nucleotide length may be at most 30 bases and preferably at most 25 bases)
- A homologous polynucleotide of LB having the base sequence of SEQ ID NO: 6: a polynucleotide comprising at least 14 (14 to 19), more preferably 16 (16 to 19) contiguous nucleotides of SEQ ID NO: 6 (in which one or several mutations may be introduced) (wherein the nucleotide length may be at most 30 bases, preferably at most 25 bases, and more preferably at most 22 bases)
- A homologous polynucleotide of FIP having the base sequence of SEQ ID NO: 7: a polynucleotide comprising at least 39 (39 to 45), more preferably at least 41 (41 to 45) contiguous nucleotides of SEQ ID NO: 7 (in which one or several mutations may be introduced) (wherein the nucleotide length may be at most 60 bases, preferably at most 52 bases, and more preferably at most 46 bases)
- A homologous polynucleotide of F3 having the base sequence of SEQ ID NO: 8: a polynucleotide comprising at least 12 (12 to 16), more preferably 14 (14 to 16) contiguous nucleotides of SEQ ID NO: 8 (in which one or several mutations may be introduced) (wherein the nucleotide length may be at most 30 bases, preferably at most 25 bases, more preferably at most 22 bases, and particularly preferably at most 20 bases)
- A homologous polynucleotide of BIP having the base sequence of SEQ ID NO: 9: a polynucleotide comprising at least 42 (42 to 51), more preferably at least 46 (46 to 51) contiguous nucleotides of SEQ ID NO: 9 (in which one or several mutations may be introduced) (wherein the nucleotide length may be at most 60 bases and preferably at most 52 bases)
- A homologous polynucleotide of B3 having the base sequence of SEQ ID NO: 10: a polynucleotide comprising at least 14 (14 to 19), more preferably 16 (16 to 19) contiguous nucleotides of SEQ ID NO: 10 (in which one or several mutations may be introduced) (wherein the nucleotide length may be at most 30 bases, preferably at most 25 bases, more preferably at most 22 bases, and particularly preferably at most 20 bases)
- A homologous polynucleotide of LF having the base sequence of SEQ ID NO: 11: a polynucleotide comprising at least 14 (14 to 19), more preferably 16 (16 to 19) contiguous nucleotides of SEQ ID NO: 11 (in which one or several mutations may be introduced) (wherein the nucleotide length may be at most 30 bases, preferably at most 25 bases, more preferably at most 22 bases, and particularly preferably at most 20 bases)
- A homologous polynucleotide of FIP having the base sequence of SEQ ID NO: 12: a polynucleotide comprising at least 33 (33 to 39), more preferably at least 36 (36 to 39) contiguous nucleotides of SEQ ID NO: 12 (in which one or several mutations may be introduced) (wherein the nucleotide length may be at most 60 bases, preferably at most 52 bases, more preferably at most 46 bases, and particularly preferably at most 42 bases)
- A homologous polynucleotide of F3 having the base sequence of SEQ ID NO: 13: a polynucleotide comprising at least 16 (16 to 20), more preferably 18 (18 to 20) contiguous nucleotides of SEQ ID NO: 13 (in which one or several mutations may be introduced) (wherein the nucleotide length may be at most 30 bases, preferably at most 25 bases, and more preferably at most 22 bases)
- A homologous polynucleotide of BIP having the base sequence of SEQ ID NO: 14: a polynucleotide comprising at least 39 (39 to 46), more preferably at least 42 (42 to 46) contiguous nucleotides of SEQ ID NO: 14 (in which one or several mutations may be introduced) (wherein the nucleotide length may be at most 60 bases and preferably at most 52 bases)
- A homologous polynucleotide of B3 having the base sequence of SEQ ID NO: 15: a polynucleotide comprising at least 16 (16 to 21), more preferably 19 (19 to 21) contiguous nucleotides of SEQ ID NO: 15 (in which one or several mutations may be introduced) (wherein the nucleotide length may be at most 30 bases, preferably at most 25 bases, and more preferably at most 22 bases)
- A homologous polynucleotide of LB having the base sequence of SEQ ID NO: 16: a polynucleotide comprising at least 16 (16 to 22), more preferably 19 (19 to 22) contiguous nucleotides of SEQ ID NO: 16 (in which one or several mutations may be introduced) (wherein the nucleotide length may be at most 30 bases and preferably at most 25 bases)
- A homologous polynucleotide of FIP having the base sequence of SEQ ID NO: 17: a polynucleotide comprising at least 39 (39 to 46), more preferably at least 42 (42 to 46) contiguous nucleotides of SEQ ID NO: 17 (in which one or several mutations may be introduced) (wherein the nucleotide length may be at most 60 bases and preferably at most 52 bases)
- A homologous polynucleotide of F3 having the base sequence of SEQ ID NO: 18: a polynucleotide comprising at least 16 (16 to 20), more preferably 18 (18 to 20) contiguous nucleotides of SEQ ID NO: 18 (in which one or several mutations may be introduced) (wherein the nucleotide length may be at most 30 bases, preferably at most 25 bases, and more preferably at most 22 bases)
- A homologous polynucleotide of BIP having the base sequence of SEQ ID NO: 19: a polynucleotide comprising at least 42 (42 to 52), more preferably at least 47 (47 to 52) contiguous nucleotides of SEQ ID NO: 19 (in which one or several mutations may be introduced) (wherein the nucleotide length may be at most 60 bases)
- A homologous polynucleotides of B3 having the base sequence of SEQ ID NO: 20: a polynucleotide comprising at least 16 (16 to 22), more preferably 18 (18 to 22) contiguous nucleotides of SEQ ID NO: 20 (in which one or several mutations may be introduced) (wherein the nucleotide length may be at most 30 bases and preferably at most 25 bases)
- A homologous polynucleotide of LF having the base sequence of SEQ ID NO: 21: a polynucleotide comprising at least 16 (16 to 21), more preferably 18 (18 to 21) contiguous nucleotides of SEQ ID NO: 21 (in which one or several mutations may be introduced) (wherein the nucleotide length may be at most 30 bases, preferably at most 25 bases, and more preferably at most 22 bases)

Such a homologous polynucleotide may be a polynucleotide having at least 90% and preferably at least 95% identity with each corresponding base sequence. The numerical value of identity can be calculated in accordance with the algorithm well known in the art field. For example, the numerical value of identity can be calculated using BLAST (http://www.ddbj.nig.ac.jp/search/blast-j.html).

Moreover, the homologous polynucleotide may be a polynucleotide that consists of a modified base sequence in which one or several mutations are introduced with respect to each of the base sequences of SEQ ID NOS: 1 to 21, and hybridizes with a polynucleotide having a sequence complementary to each of the base sequences of SEQ ID NOS: 1 to 21.

Herein, the term "mutations", which may be the same or different, may be selected from a substitution, a deletion, an insertion and an addition. Such mutation may be preferably selected from "one base substitution" in which a certain base is substituted with another base, "one base deletion" in which a certain base is deleted, "one base insertion" in which a certain base is inserted, and "one base addiction" in which a certain base is added. The number of mutations may be 1 to 6 bases, 1, 2, 3 or 4 bases, 1 or 2 bases, or 1 base.

In the present invention, the term "polynucleotide" means to include DNA, RNA and PNA (peptide nucleic acid).

A polynucleotide that constitutes the primer set according to the present invention may be prepared by the chemical synthesis of a nucleic acid according to an ordinary method such as a phosphate triester method (Hunkapiller, M. et al., Nature, 310, 105, 1984). Otherwise, the total DNA of a strain as a detection target may be obtained, and a DNA fragment containing a nucleotide sequence of interest may be then obtained, as appropriate, by the PCR method or the like, based on the nucleotide sequences disclosed in the present specification.

More specific embodiments of the detection method according to the present invention may include methods for detecting the yeast of genus Dekkera and the yeast of genus Brettanomyces, which comprise performing a nucleic acid amplification reaction on a nucleic acid sample by a LAMP method and then detecting the presence or absence of a nucleic acid amplification product. Specifically, there are provided the following methods.

In the first embodiment according to the present invention, there is provided a method for detecting Dekkera anomala, which comprises:
(a) performing a nucleic acid amplification reaction on a nucleic acid contained in a sample by a LAMP method using a primer set consisting of polynucleotides 1, 2, 3 and 4; and
(b) detecting the presence or absence of an amplification product,
wherein the generation of the amplification product indicates the presence of Dekkera anomala.

In the second embodiment according to the present invention, there is provided a method for detecting Dekkera bruxellensis, which comprises
(c) performing a nucleic acid amplification reaction on a nucleic acid contained in a sample by a LAMP method using a primer set consisting of polynucleotides 7, 8, 9 and 10; and
(d) detecting the presence or absence of an amplification product,
wherein the generation of the amplification product indicates the presence of Dekkera bruxellensis.

In the third embodiment according to the present invention, there is provided a method for detecting Dekkera custersiana, which comprises
(e) performing a nucleic acid amplification reaction on a nucleic acid contained in a sample by a LAMP method using a primer set consisting of polynucleotides 12, 13, 14 and 15; and
(f) detecting the presence or absence of an amplification product,
wherein the generation of the amplification product indicates the presence of Dekkera custersiana.

In the fourth embodiment according to the present invention, there is provided a method for detecting Brettanomyces naardenensis, which comprises
(g) performing a nucleic acid amplification reaction on a nucleic acid contained in a sample by a LAMP method using a primer set consisting of polynucleotides 17, 18, 19 and 20; and
(h) detecting the presence or absence of an amplification product,
wherein the generation of the amplification product indicates the presence of Brettanomyces naardenensis.

A sample subjected to the nucleic acid amplification process by the LAMP method may be prepared in such a way that cells contained in the sample are cultured, and a nucleic acid is extracted from the cultured cells, or such a nucleic acid is extracted without such a culture process. The preparation of a nucleic acid sample, such as the culture of cells, the extraction of a nucleic acid will be described later.

In the nucleic acid amplification process by the LAMP method, an amplification reaction is performed on a nucleic acid contained in a sample. Such a nucleic acid amplification reaction by the LAMP method will be described later.

In a case where a detection target species exists in a sample, a specific region as a target is amplified, and an amplification product is generated. When such an amplification product is generated, a sample solution subjected to a nucleic acid amplification reaction becomes clouded. Thus, the presence or absence of the amplification product can be determined by measuring the turbidity of the sample solution. The measurement of the turbidity by the LAMP method is well known. The turbidity can be measured using a commercially available end point turbidity measurement apparatus (e.g. LA-100 manufactured by Teramecs Co., Ltd.) or a real-time turbidity measurement apparatus (e.g. LA-200 manufactured by Teramecs Co., Ltd.).

As described in the examples as given later, a time required until a sample solution reaches a certain turbidity is measured, so as to determine the number of cells contained in a test sample. Specifically, in another aspect of the detection method according to the present invention, there is provided a method for quantifying the yeast of genus Dekkera and the yeast of genus Brettanomyces, which comprises performing a nucleic acid amplification reaction on a nucleic acid sample by the LAMP method, and at the same time, measuring a time required from the initiation of the nucleic acid amplification reaction until a sample solution reaches a certain turbidity and obtaining the number of cells contained in the sample from the measured time. The quantification method of the present invention is specifically as follows.

In the first embodiment according to the present invention, there is provided a method for quantifying Dekkera anomala, which comprises:
(a) performing a nucleic acid amplification reaction on a nucleic acid contained in a sample by a LAMP method using a primer set consisting of polynucleotides 1, 2, 3 and 4;
(b') measuring a time required from the initiation of the nucleic acid amplification reaction until a sample solution reaches a certain turbidity; and
(b") obtaining the number of cells contained in the sample from the measured time.

In the second embodiment according to the present invention, there is preferably provided a method for quantifying Dekkera bruxellensis, which comprises:
(c) performing a nucleic acid amplification reaction on a nucleic acid contained in a sample by a LAMP method using a primer set consisting of polynucleotides 7, 8, 9 and 10;
(d') measuring a time required from the initiation of the nucleic acid amplification reaction until a sample solution reaches a certain turbidity; and
(d") obtaining the number of cells contained in the sample from the measured time.

In the third embodiment according to the present invention, there is preferably provided a method for quantifying Dekkera custersiana, which comprises:
(e) performing a nucleic acid amplification reaction on a nucleic acid contained in a sample by a LAMP method using a primer set consisting of polynucleotides 12, 13, 14 and 15;
(f') measuring a time required from the initiation of the nucleic acid amplification reaction until a sample solution reaches a certain turbidity; and
(f") obtaining the number of cells contained in the sample from the measured time.

In the fourth embodiment according to the present invention, there is preferably provided a method for quantifying Brettanomyces naardenensis, which comprises:
(g) performing a nucleic acid amplification reaction on a nucleic acid contained in a sample by a LAMP method using a primer set consisting of polynucleotides 17, 18, 19 and 20;
(h') measuring a time required from the initiation of the nucleic acid amplification reaction until a sample solution reaches a certain turbidity; and
(h") obtaining the number of cells contained in the sample from the measured time.

In the quantification method according to the present invention, a calibration curve has previously been produced using the number of cells and a time required until a sample solution reaches a certain turbidity. Thereafter, the number of cells contained in the sample can be obtained from the measured time based on the calibration curve. The calibration curve can be produced, for example, by preparing samples by stepwisely diluting cells, then performing a nucleic acid amplification method on each sample according to the LAMP method, and then plotting a time required from the initiation of the nucleic amplification reaction until the turbidity becomes 0.1 with respect to the logarithm of the colony formation number of cells.

In the detection method and the quantification method according to the present invention, a Loop primer(s) (LF and/or LB) may be added to a primer set consisting of FIP, F3, BIP and B3, and a nucleic acid amplification reaction may be then carried out by the LAMP method. Specifically, in the detection method and quantification method of the first embodiment according to the present invention, any one or both of polynucleotide 5 and polynucleotide 6 may be added as Loop primer(s) and may be used. In the detection method and quantification method of the second embodiment according to the present invention, polynucleotide 11 may be added as a Loop primer and may be used. In the detection method and quantification method of the third embodiment according to the present invention, polynucleotide 16 may be added as a Loop primer and may be used. In the detection method and quantification method of the fourth embodiment according to the present invention, polynucleotide 21 may be added as a Loop primer and may be used.

The primer set according to the present invention can be provided in the form of a kit, singly or in combination. Thus, according to the present invention, there is provided a kit for detecting the yeast of genus Dekkera and/or the yeast of genus Brettanomyces, which comprises a primer set selected from the group consisting of the primer set of the first embodiment; the primer set of the second embodiment; the primer set of the third embodiment; the primer set of the fourth embodiment; and a combination of these primer sets.

The kit according to the present invention may comprise reagents (for example, Bst DNA polymerase, a reagent mixed solution for reaction) and apparatuses (for example, a reaction tube), which are necessary for the implementation of the nucleic acid amplification reaction by the LAMP method.

If the nucleic acid amplification reaction is carried out by the LAMP method using the primer set according to the present invention, the yeast of genus Dekkera and the yeast of genus Brettanomyces, which cause a decrease in the quality of alcoholic beverages or soft drinks, can be accurately identified at a species level, and at the same time, these yeasts can be detected with high sensitivity. Accordingly, the primer set and kit according to the present invention can be used in the quality control of alcoholic beverages (for example, beer, low-malt beer, wine, fruit wine) and/or soft drinks (for example, nonalcoholic sparkling drinks such as lemon soda and carbonated water) the examination of an environmental sample (for example, raw material water).

Dekkera anomala, Dekkera bruxellensis and Dekkera custersiana, may be found as yeast species causing quality deterioration in the production process of beer and low-malt beer or in the final products thereof. Therefore, the primer set of the first embodiment, the primer set of the second embodiment, the primer set of the third embodiment, the combined use thereof, detection and quantification methods using any one of these primers, and a kit comprising any one of these primer sets can be preferably used in the quality control of beer and low-malt beer.

Dekkera bruxellensis may be found as a yeast species causing quality deterioration in a wine production process or in the final product thereof. Therefore, the primer set of the second embodiment, detection and quantification methods using this primer set, and a kit comprising this primer set can be preferably used in the quality control of wine.

Brettanomyces naardenensis may be found as a yeast species causing quality deterioration in the production process of soft drinks (in particular, soda water and lemonade) or in the final products thereof. Therefore, the primer set of the fourth embodiment, detection and quantification methods using this primer set, and a kit comprising this primer set can be preferably used in the quality control of soft drinks (in particular, soda water and lemonade).

### Nucleic acid amplification reaction by LAMP method

The primer set according to the present invention can be used as primers for a nucleic acid amplification reaction by a LAMP method. The primer set according to the present invention can also be used as primers not only for the nucleic acid amplification reaction by the LAMP method, but also for a nucleic acid amplification reaction by a modified LAMP method. The principle of the LAMP method and a nucleic acid amplification method utilizing it are well known. In order to carry out the nucleic acid amplification reaction by the LAMP method, descriptions disclosed in WO00/28082, and Notomi T. et al., Nucleic Acids Research, 28(12), e63(2000) can be used as references.

The nucleic acid amplification reaction by the LAMP method can be carried out using a commercially available LAMP method gene amplification reagent kit. The nucleic acid amplification reaction can be carried out, for example, by mixing sample DNA, a primer solution, and reagents included with a commercially available LAMP method gene amplification reagent kit (for example, a Loopamp DNA amplification kit manufactured by Eiken Chemical Co., Ltd.) in accordance with instructions included with the kit, and then retaining the obtained mixture at a certain temperature (60°C to 65°C) so as to react it for a certain period of time (in general, 1 hour).

The nucleic acid amplification reaction by the LAMP method can be carried out via the following processes.
(i) A DNA strand complementary to template DNA is synthesized by the action of strand displacement-type DNA polymerase, using the 3'-terminus of the F2 region of FIP as an origin.
(ii) An F3 primer is annealed to a site outside the FIP, and DNA synthesis is extended by the action of the strand displacement-type DNA polymerase, using the 3'-terminus thereof as an origin, while removing the previously synthesized DNA strand from the FIP.
(iii) A double strand is formed by a DNA strand synthesized from the F3 primer and the template DNA.
(iv) The DNA strand previously synthesized from the FIP is removed due to the DNA strand from the F3 primer, so that it becomes single-stranded DNA. However, this DNA strand has complementary regions F1c and F1 on the 5'-terminal side, and it causes self-annealing so as to form a loop.
(v) BIP is annealed to the DNA strand that has formed a loop in the process of (iv) above, and complementary DNA is synthesized using the 3'-terminus of the BIP as an origin. In this process, the loop is removed and extended. Further, a B3 primer is annealed to a site outside the BIP, and DNA synthesis is extended by the action of the strand displacement-type DNA polymerase, using the 3'-terminus thereof as an origin, while removing the previously synthesized DNA strand from the BIP.
(vi) Double-stranded DNA is formed in the process of (v) above.
(vii) Since the DNA strand synthesized from the BIP that has been removed in the process of (v) above has complementary sequences on both termini, it causes self-annealing to form a loop, so that it has a dumbbell-like structure.
(viii) Using the aforementioned DNA strand having a dumbbell-like structure as an origin, an amplification cycle of desired DNA is carried out via the annealing of the FIP and then the annealing of the BIP.

It would be obvious to those skilled in the art to carry out the nucleic acid amplification reaction by the LAMP method by appropriately modifying the aforementioned processes. The primer set according to the present invention can also be used in such a modified method.

The primer set according to the present invention brings on the synthesis of a DNA strand at a temperature of approximately 60°C to approximately 65°C (for example, 65°C), as well as annealing. A reaction is carried out for approximately 1 hour via the annealing reaction and the DNA strand synthesis, so that a nucleic acid can be amplified by 10⁹ to 10¹⁰ times.

If the primer set according to the present invention is allowed to react with a sample nucleic acid under conditions for the nucleic acid amplification reaction by the LAMP method, a target region in the ITS region of a strain as a detection target is amplified. When such an amplification reaction takes place, a reaction solution becomes clouded due to the influence of magnesium pyrophosphate formed as a by-product. Thus, based on the turbidity, the presence or absence of amplification can be determined by visual observation. The presence or absence of amplification may also be determined by optically measuring the turbidity using a turbidity measurement apparatus. Alternatively, agarose gel electrophoresis or the like may be applies to confirm and detect the presence or absence of a DNA fragment.

If nucleic acid amplification is observed, it means that a target gene is present, indicating that the strain as a detection target of the primer set is positive (+). To the contrary, if such nucleic acid amplification is not observed, it means that a target gene is absent, indicating that the strain as a detection target of the primer set is negative (-).

### Nucleic acid samples and preparation thereof

Examples of a sample used as a detection target of the primer set and kit according to the present invention include: alcoholic beverages such as beer, low-malt beer and wine; soft drinks such as cider, lemon soda and carbonated water; environmental samples such as water collected for use as a raw material; and half-finished products collected from the production process of alcoholic beverages, soft drinks, etc.

When these products are used as samples for the LAMP method, operations such as the concentration, separation and culture of cells existing in the sample, the separation of a nucleic acid from the cells, and the concentration of the nucleic acid may be carried out as pre-treatments. Methods for concentration and separation of cells existing in the sample include filtration and centrifugation, and such methods can be selected, as appropriate. In addition, the cells concentrated and separated from the sample may be further cultured, so as to increase the number of the cells. For the culture, an agar solid medium or a liquid medium, which is suitable for the proliferation of the target yeast strain, may be used. Moreover, in order to select the target yeast strain, an agent such as cycloheximide may be added. In order to release a nucleic acid from cells existing in a beverage sample or an environmental sample or from the cultured cells, a method using a commercially available kit or a method of treating cells with an alkali solution and then heating the cells at 100°C to release a nucleic acid from them can be selected, for example. Furthermore, if it is necessary to further purify a nucleic acid, the nucleic acid may be purified by a phenol/chloroform treatment, ethanol precipitation, centrifugation, etc., and the purified nucleic acid may be finally re-dissolved in a TE buffer or the like, so that it may be used as template DNA in tests (European Brewery Convention: ANALYTICA-MICROBIOLOGICA-EBC, 2nd ed. 2005, Fachverlag Hans Carl, Nuernberg; Rolf et al.: PCR-Clinical diagnostics and research, Springer-Veriag, Berlin, 1992; Yasuji Oshima et al.: Tanpaku kakusan koso (Proteins, Nucleic acids, Enzymes), Vol. 35, 2523-2541, 1990).

The detection of the yeast of genus Dekkera and the yeast of genus Brettanomyces using the primer set and kit according to the present invention can be carried out as follows, for example.

First, the yeast of genus Dekkera and the yeast of genus Brettanomsrces that are considered to exist in a sample are cultured in a suitable medium. Next, DNA is separated from a colony formed on the agar medium, and the LAMP method using the primer set according to the present invention is then applied to the DNA, so as to amplify the specific gene regions of the yeast of genus Dekkera and the yeast of genus Brettanomyces. The obtained gene amplification product indicates the presence of a strain as a target of the primer set.

The "genus Dekkera" is a designation of the sexual generation of the "genus Brettanomyces." In taxonomy, it is possible that a single type of yeast has different designations for its sexual generation and asexual generation, and thus the two designations are consistent with each other. That is, needless to say, the primer sets of the first, second and third embodiments of the present invention can also be used in the detection of the strain of the corresponding genus Brettanomyces, and the primer set of the fourth embodiment of the present invention can be used in the detection of the strain of the corresponding genus Dekkera.

### Examples

Hereinafter, the present invention will be specifically described in the following examples. However, these examples are not intended to limit the scope of the present invention.

### Example 1: Detection of east of genus Dekkera and yeast of genus Brettanomyces

### (a) Genomic DNA extraction method

Cells cultured on an agar plate medium were scraped from the medium, and they were then suspended in sterilized distilled water. This suspension was centrifuged (15,000 rpm, 5 minutes), and a supernatant was then discarded. Sterilized distilled water was added to the precipitated cells again, and the mixed solution was then suspended and centrifuged. A supernatant was discarded, and 100 µl of a solution of PrepMan Ultra (manufactured by Applied Biosystems) was then added to the obtained cells. The mixture was heated at 95°C for 10 minutes. Thereafter, the resultant was centrifuged at 15,000 rpm for 1 minute, and a supernatant was used as a genomic DNA solution. Otherwise, 100 µl of a 0.1 N NaOH solution was added to the washed cells, and the mixture was then heated at 95°C for 10 minutes. Thereafter, the resultant was neutralized with a 1 M Tris buffer (pH 7.0), and a supernatant was used as a genomic DNA solution.

### (b) Primers for use in LAMP

Primers for use in 4 types of yeast strains of genus Dekkera as described below were chemically synthesized using eGenome Order (http://genome.e-mp.jp/index.html) manufactured by Fujitsu System Solutions Ltd., and they were then dissolved in a TE buffer (pH 8.0), resulting in a concentration of 100 µM. These solutions were mixed so that they had each predetermined concentrations (FIP and BIP primers: 16 µM; F3 and B3 primers: 2 µM; and LF and LB primers: 8 µM), and they were then diluted.
[Primer set for use in detection of Dekkera anomala (DA1LF1LB1)]
FIP: ACCGCGTATTCTCATAACCACTCCGGGCATGCCTGTTTGAGC (SEQ ID NO:: 1)
F3: AGTTCTTGAACGCACATTGC (SEQ ID NO: 2) BIP: 
ACTGGGAGGAGTAATAATTACAAGGTTTCGGGGACTCAGTAGGAGTT
GCTA (SEQ ID NO: 3)
 B3: TCCTCCTACCTGATTTGAGGT (SEQ ID NO: 4)
LF: AGAGAAGTGAGAAGGAAATGAC (SEQ ID NO: 5)
LB: GCCGATTTATTGCGTCTTC (SEQ ID NO: 6)
[Primer set for use in detection of Dekkera bruxellensis
(DB1LF1)]
FIP: ACCCTCGTGTAATCTCATAACCACTAAGGAGGGCATGCCTGTTTG (SEQ ID NO: 7)
F3: ACATTGCGCCCTCTGG (SEQ ID NO: 8) BIP: 
GATTTAAGGTTTCGGCCGTTCATTATTTTCTCCTACCTGATTTGAGGTCA
A (SEQ ID NO: 9)
 B3: TGCTTAAGTTCAGCGGGTC (SEQ ID NO: 10)
LF: AGTGAGAAGGAAATGACGC (SEQ ID NO: 11)
[Primer set for use in detection of Dekkera custersiana (DC2LB1)]
FIP: CGGTGCCGTCATCACAACTACGGGCATGCCTGTTTGAGC (SEQ ID NO: 12)
F3: AGTTCTTGAACGCACATTGC (SEQ ID NO: 13) BIP:
GCGGTCCGTCGAAGAGAATGACGTAGATAAAAGTCAACGACTGGTC (SEQ ID NO: 14)
B3: TCCTCCTACCTGATTTGAGGT (SEQ ID NO: 15)
LB: AGTTAACGGAGTAAGGTTTC (SEQ ID NO: 16)
[Primer set for use in detection of Brettanomyces naardenensis (BN1LF1)]
FIP:
GAGAGGTATCTTCAAAACCACTTGCGATTCTGGGGGGTATTCCTGT (SEQ ID NO: 17)
F3: AGTTCTTGAACGCACATTGC (SEQ ID NO: 18)
BIP: 
GGAATTCTGAATTTAGTTGGCTTCTTGTTTGCTTTCAACGAATGGTCGA
AAC (SEQ ID NO: 19)
 B3: AAACGTCAAGGTCATTGACTAC (SEQ ID NO: 20)
LF: TGAGAAGGAAATGACGCTCAA (SEQ ID NO: 21)

### (c) Preparation of LAMP amplification reaction solution

A Loopamp DNA amplification kit manufactured by Eiken Chemical Co., Ltd. was used as a gene amplification reagent kit for the LAMP method. 2.5 µl of a genomic DNA solution, 2.5 µl of a primer solution, 12.5 µl of a 2-times concentration reaction buffer, 1 µl of Bst DNA polymerase, and 6.5 µl of sterilized water were added to a reaction tube, and a reaction solution in a total amount of 25 µl was thereby prepared.

### (d) LAMP reaction

A real-time turbidimeter LA-200 manufactured by Teramecs Co., Ltd. was used for a LAMP reaction. A reaction tube was set, and the reaction solution was allowed to react at a constant temperature of 65°C (bonnet: 75°C), and a change in turbidity during the reaction was measured every 6 seconds. The reaction solution whose turbidity was increased was defined as positive, and the reaction solution whose turbidity was not increased was defined as negative.

### (e) Evaluation of specificity of primer for use in each yeast strain

With regard to the 4 types of yeast strains of genus Dekkera, primers specific for each yeast strain were produced, and such primers were evaluated by the LAMP method using each yeast strain.

As a result, it was found that each primer specifically reacted with a yeast strain as a target, and that it hardly reacted with other types of yeast strains. Figure 1 shows a process in which when a primer reacted with a strain as a detection target, amplification took place within 60 minutes after the initiation of the reaction, and the turbidity in the reaction tube was increased.

Moreover, the specificity of each primer was evaluated using various types of standard yeast strains, yeast strains for use in brewing, wild-type yeast strains separated at a brewery, and wild-type yeast strains separated from wine. As a result, it was found that almost no amplification was observed from strains other than a strain used as an examination target of each primer, and that the specificity of each primer for a yeast strain as a target was extremely high (Tables 1 to 4).

Table 1: Evaluation of primers using various types of yeast strains of genus Dekkera (the number in each cell of the table: reaction time required until amplification took place; -: no amplification occurred within 60 minutes after the reaction)

**[Table 1]**

| Detection target yeast | Separation source | DA1LF1LB1 | DB1LF1 | DC2LB1 | BN1LF1 |
|---|---|---|---|---|---|
| Dekkera anomala DSM70727 | Stout beer | 20 min | - | - | - |
| D. anomala ATCC56868 | Cider | 20 min | - | - | - |
| D. anomala ATCC58984 | Deteriorated soft drink | 20min | - | - | - |
| D. bruxellensis DSM70001 | Lambic beer | - | 30 min | - | - |
| D. bruxellensis ATCC64276 | Deteriorated red wine | - | 30 min | - | - |
| D. custersiana DSM70736 | Brewery | - | - | 40 min | - |
| Brettanomyces naardenensis NBRC1588 | Lemonade | - | - | - | 40 min |
| B. naardenensis ATCC56870 | Carbonated water | - | - | - | 30 min |
| D. bruxellensis WY96 | Brewery | - | 30 min | - | - |
| D. bruxellensis WY97 | Brewery | - | 30 min | - | - |
| D. bruxellensis WY98 | Brewery | - | 30 min | - | - |

Table 2: Evaluation of primers using various types of standard yeast strains (the number in each cell of the table: reaction time required until amplification took place; -: no amplification occurred within 60 minutes after the reaction)

**[Table 2]**

| Standard yeast strain | DA1LF1LB1 | DBLLF1 | DC2LB1 | BN1LF1 |
|---|---|---|---|---|
| Saccharomyces cerevisiae NBRC10217 | - | - | - | - |
| S. bayanus NBRC11022 | - | - | - | - |
| S. pastorianus CBS1538 | - | - | - | - |
| S. pastorianus NBRC11023 | - | - | - | - |
| S. pastorianus NBRC10610 | - | - | - | - |
| S. diastaticus DSM70487 | - | - | - | - |
| S. paradoxus NBRC10609 | - | - | - | - |
| S. cariocanus NBRC10941 | - | - | - | - |
| S. mikatae NBRC1815 | - | - | - | - |
| S. kudriavzevii NBRC1802 | - | - | - | - |
| S. exiguus NBRC1128 | - | - | - | - |
| S. servazzi MBRC1838 | - | - | - | - |
| S. unisporus NBRC0316 | - | - | - | - |
| S. dairenensis NBRC0211 | - | - | - | - |
| S. kluyveri NBRC1685 | - | - | - | - |
| Pichia anomala NBRC0127 | - | - | - | - |
| Williopsis saturnus NBRC0941 | - | - | - | - |
| Kluyveromyces lactis NBRC1090 | - | - | - | - |
| Candida utilis NBRC0988 | - | - | - | - |
| C. boidinii ATCC48180 | - | - | - | - |
| Zygosaccharomyces bailii NBRC1137 | - | - | - | - |

Table 3: Evaluation of primers using various types of yeast strains for use in brewing and wild-type yeast strains separated at brewery (the number in each cell of the table: reaction time required until amplification took place; -: no amplification occurred within 60 minutes after the reaction)

**[Table 3]**

| Brewing yeast and wild-type beer yeast | DA1LF1LB1 | DB1LF1 | DC2LB1 | BN1LF1 |
|---|---|---|---|---|
| Bottom fermentation yeast BY61 | - | - | - | - |
| Bottom fermentation yeast BFY70 | - | - | - | - |
| Bottom fermentation yeast BFY84 | - | - | - | - |
| Top fermentation yeast TFY3 | - | - | - | - |
| Top fermentation yeast TFY23 | - | - | - | - |
| Trichosporon cutaneum WY54 | - | - | - | - |
| C. intermedia WY55-1 | - | - | - | - |
| Debaryomyces hansenii WY69 | - | - | - | - |
| P. membranifaciens WY75 | - | - | - | - |
| Rhodotorula graminis WY93 | - | - | - | - |
| S. cerevisiae WY101 | - | - | - | - |
| Pichia sp. WY102 | - | - | - | - |
| Kluyveromyces sp. WY114 | - | - | - | - |
| S. diastaticus WY126 | - | - | - | - |

Table 4: Evaluation of primers using yeast strains separated from various types of wines (the number in each cell of the table: reaction time required until amplification took place; -: no amplification occurred within 60 minutes after the reaction)

**[Table 4]**

| Wild-type wine yeast | DA1LF1LB1 | DB1LF1 | DC2LB1 | BN1LF1 |
|---|---|---|---|---|
| Z. bailii WLY9 | - | - | - | - |
| S. cerevisiae WLY10 | - | - | - | - |
| P. membranifaciens WLY13 | - | - | - | - |
| Lodderomyces elongisporus WLY14 | - | - | - | - |
| Aureobasidium pullulans WLY15 | - | - | - | - |
| Rhodosporidium fluviale WLY16 | - | - | - | - |
| P. anomala WLY17 | - | - | - | - |
| P. guilliermondii WLY18 | - | - | - | - |

The above results demonstrated that the primer sets according to the present invention produced for the 4 types of yeast strains are able to accurately detect the yeast of genus Dekkera and the yeast of genus Brettanomyces at a species level.

### Example 2: Comparison of detection limit of LAMP method with detection limit of PCR method

In order to compare the amplification efficiency of the LAMP method with that of the conventional PCR method, the cells of Dekkera bruxellensis WY97 cultured on an agar plate medium were stepwisely diluted, and DNA was then extracted by the aforementioned method. The extracted DNA was subjected to the LAMP method and the PCR method.

Ex Taq manufactured by Takara Bio Inc. was used for the PCR method. The base sequences of primers for use in the detection of Dekkera bruxellensis, which were used in PCR, are as follows.
Sense primer: GATAAAAATACATTAAATTTATTTAGTT (SEQ ID NO: 22)
Antisense primer: GAACGGCCGAAACCTTAAATC (SEQ ID NO: 23)

The antisense primer of this primer set has the same region as the sequence used to design the LAMP primer. Sequences specific for yeast strains used in the present specification can also be used in other gene amplification techniques. These reagents were mixed in accordance with the instruction of the Ex Taq manufactured by Takara Bio Inc., and the mixture was then set in a thermal cycler, A temperature program of 94°C-30 seconds, 50°C-30 seconds, and 72°C-1 minute was repeated 25 times, so as to carry out a PCR reaction.

As a result, amplification was observed even from cells in an amount of a 10¹ cfu level (1.8 × 10¹ cfu) in the LAMP method, but in the PCR method, no bands were observed from cells in an amount lower than a 10⁴ cfu level (1.8 × 10⁴ cfu) (Figures 2 and 3). Thus, in a method for detecting the yeast of genus Dekkera by the LAMP method, a gene can be amplified from cells in a smaller amount, even using the same DNA. Accordingly, it can be said that the detection sensitivity of the detection method involving the LAMP method is extremely higher than that of the conventional PCR method.

Even in the PCR method, if the number of cycles is increased, such detection sensitivity can be increased. In such a case, however, a time at which the reagents are set in the thermal cycler becomes longer. In the case of the PCR method, such a time necessary for the reaction in the thermal cycler is approximately 1.5 hours, and the results cannot be confirmed unless electrophoresis and DNA staining are performed. In the detection method involving the LAMP method, even if the amount of cells was small, detection could be completed within 60 minutes after the initiation of the reaction, and thus it was found that the detection method involving the LAMP method is more advantageous than the PCR method in terms of amplification efficiency and operation time.

With regard to LAMP primers other than DB1LF1, the detection limit of each primer to a yeast strain used as a target of the primer in the detection method involving the LAMP method was analyzed. As a result, it was found that DA1LF1LB1, DC2LB1 and BN1LF1 also have a detection sensitivity of approximately 1 × 10¹ cfu, as with DB1LF1.

The detection limit of each primer in the case of suspending cells in sterilized water is as follows.
DA1LF1LB1: 9.8 × 10⁰ cfu
DB1LF1: 1.8 × 10¹ cfu
DC2LB1: 9.3 × 10⁰ cfu
BN1LF1: 6.3 × 10¹ cfu

In addition, a nucleic acid amplification reaction according to the LAMP method was performed on genomic DNA extracted from the diluted solution of cells at each stage. A time required from the initiation of the reaction until the turbidity of the reaction solution exceeded 0.1 was measured. A graph was formed from a logarithm of a time in the case of using DB1LF1, DA1LF1LB1, DC2LB1 and BN1LF1 (threshold time) and the number of colony formation. As a result of exponential approximation, an approximate curve of high correlation coefficient could be formed (R² = 0.971 - 0.999). The approximate curve of DA1LF1LB1 is as shown in Figure 4.

As described above, it was demonstrated that, by forming a calibration curve of a time required until the reaction solution reached a certain turbidity and the colony formation number of cells, the yeast of genus Dekkera and the yeast of genus Brettanomyces contained in a sample can be quantitatively measured within the range from 1 × 10¹ cfu to 1 × 10⁷ cfu.

### Example 3: Direct detection of cells in wine and beer

In order to analyze whether or not the yeast of genus Dekkera can be detected from wine, Dekkera bruxellensis (ATCC64276) was cultured on a malt agar medium. Thereafter, the resultant cells were suspended in red wine sterilized with a filter, and they were then stepwisely diluted using the same red wine. This diluted solution of cells and red wine was centrifuged, and a supernatant was then discarded to obtain a precipitate. An alkali solution was added to this precipitate by the method of the aforementioned example, and DNA was then extracted. From this extract, a phenomenon whereby amplification did not occur in the LAMP reaction or a time required for the initiation of amplification was retarded for 10 minutes or more took place. It was considered that red wine remaining in the centrifuge tube inhibited the LAMP reaction.

The cell-red wine diluted solution prepared in the same above manner was centrifuged, and a supernatant was discarded. 1 ml of sterilized water was added thereto to wash the precipitate. Thereafter, a precipitate was obtained by centrifugation again, and DNA was then extracted. As a result, it became possible to detect cells by the LAMP reaction. Thus, it was found that when the presence of cells was directly detected from beverages such as wine by the LAMP reaction, the collection and washing of the cells were necessary. It was found that when the cells were collected and washed, the detection limit of the cells suspended in wine was at the same level as that when the cells were suspended in sterilized water. Moreover, when Dekkera bruxellensis WY97 was suspended in beer as well, a similar detection limit was obtained.

When Dekkera bruxellensis was suspended in wine, beer and sterilized water, its detection limit is as follows (cell collection and washing was carried out once in the case of wine and beer samples).
Sterilized water: 1.8 × 10¹ cfu
Wine: 1.5 × 10¹ cfu
Beer: 5.4 × 10¹ cfu

In the production of wine, in general, a yeast of genus Saccharomyces used as a wine yeast is added to fruit juice for fermentation. Since the yeast of genus Dekkera contaminates fruit juice from outside, the number of yeast cells of genus Dekkera is significantly smaller than that of the yeast of genus Saccharomyces. However, even if the number of cells is small, if the yeast of genus Dekkera exists, such yeast of genus Dekkera proliferates in the second half of fermentation and during storage, so that it influences on the quality of wine. Accordingly, early detection of the yeast of genus Dekkera is important. Thus, 1 × 10⁷ cells of Saccharomyces cerevisiae were suspended in wine, and thereafter, the cell diluted solution of Dekkera bruxellensis (ATCC64276) as prepared by the aforementioned method was mixed therewith, followed by cell collection and washing. Thereafter, the cells were gathered, and DNA was then extracted therefrom. Thus, whether or not the yeast of genus Dekkera can be detected by the LAMP method was analyzed.

As a result, it was found that, regardless of the presence of the yeast of genus Saccharomyces, the yeast of genus Dekkera could be detected at the same detection limit. Likewise, the bottom fermentation yeast was suspended in beer, and a cell diluted solution of Dekkera bruxellensis WY97 was then mixed therewith. In this case also, the same results were obtained.

The detection limit of Dekkera bruxellensis in the case of adding the yeast of genus Saccharomyces to each of wine and beer is as follows.
Wine + Saccharomyces cerevisiae (1 × 10⁷ cells): 1.5 × 10¹ cfu
Beer + bottom fermentation yeast (1 × 10⁷ cells): 5.4 × 10¹ cfu

### Example 4: Comparison regarding detection primers

### (a) Comparison regarding primers for use in the detection of Dekkera custersiana

A primer set for use in the detection of Dekkera custersiana (DC2) and a control primer set (DC1) as mentioned below were chemically synthesized in the same manner as that of Example 1(b), and at the same time, primer solutions for use in the LAMP method were prepared. The control primer set (DC1) targeted for an ITS region (ITS1) that was different from the ITS region (ITS2) as a target of the primer set according to the present invention (DC2). In addition, ITS2 corresponds to a region between a 5.8S rRNA gene and a 26S rRNA gene (large subunit).
[Primer set for use in detection of Dekkera custersiana (DC2)]
FIP: CGGTGCCGTCATCACAACTACGGGCATGCCTGTTTGAGC (SEQ ID NO: 12)
F3: AGTTCTTGAACGCACATTGC (SEQ ID NO: 13)
BIP:
GCGGTCCGTCGAAGAGAATGACGTAGATAAAAGTCAACGACTGGTC (SEQ ID NO: 14)
B3: TCCTCCTACCTGATTTGAGGT (SEQ ID NO: 15)
[Control primer set (DC1)]
F3:AACAAGGTTTCCGTAGGTGA (SEQ ID NO: 24)
B3:AATTCACATTAGTTATCGCAATTCG (SEQ ID NO: 25)

FIP:CATGTGTTATAATGCTTGCACTCGGCTGCGGAAGGATCATTAACG
A (SEQ ID NO: 26)

BIP:GCATACAAACACAACAAACCAAAATTTATCAACTTCATCGACGCG
AGAACCA (SEQ ID NO: 27)

In accordance with the descriptions of Examples 1(c) and (d), various types of yeast strains were detected by the LAMP method using the aforementioned primer sets. The results are as shown in Figures 5 and 6.

In the case of using the primer set (DC2) according to the present invention, a target strain could be detected in a short time (Figure 5). However, in the case of using the control primer set (DC1), an extremely long period of time was required for the detection of the target strain (Figure 6). A Loop primer was added to the control primer set (DC1), and the same detection test was then carried out. As a result, a time required for an increase in the turbidity was reduced, but when compared with the primer set according to the present invention (DC2), there was still a difference for approximately 10 minutes (data not shown).

Thus, it was demonstrated that, when compared with a primer set targeting for another ITS region, the primer set according to the present invention has extremely high detection reactivity.

### (b) Comparison regarding primers for use in the detection of Dekkera anomala

A primer set for use in the detection of Dekkera anomala (DA1LF1LB1) and a control primer set (DA2LF1LB1) as mentioned below were chemically synthesized according to an ordinary method, and primer solutions for use in the LAMP method were prepared in the same manner as that of Example 1(b). FIP, F3 and LF of the control primer set (DA2LF1LB1) were the same as those of the primer set according to the present invention (DA1LF1LB1). However, the designing positions of BIP, B3 and LB were somewhat different from those of the primer set according to the present invention (DA1LF1LB1).
[Primer set for use in detection of Dekkera anomala (DA1LF1LB1)]
FIP: ACCGCGTATTCTCATAACCACTCCGGGCATGCCTGTTTGAGC (SEQ ID NO: 1)
F3: AGTTCTTGAACGCACATTGC (SEQ ID NO: 2)
BIP: 
ACTGGGAGGAGTAATAATTACAAGGTTTCGGGGACTCAGTAGGAGTT
GCTA (SEQ ID NO: 3)
 B3: TCCTCCTACCTGATTTGAGGT (SEQ ID NO: 4)
LF: AGAGAAGTGAGAAGGAAATGAC (SEQ ID NO: 5)
LB: GCCGATTTATTGCGTCTTC (SEQ ID NO: 6)
[Control primer set (DA2LF1LB1)]
FIP:ACCGCGTATTCTCATAACCACTCC-GGGCATGCCTGTTTGAGC (SEQ ID NO: 1)
F3:AGTTCTTGAACGCACATTGC (SEQ ID NO: 2)
BIP:CCGGTGGGGAGTATACTGGGAGTAGGAGTTGCTAGAAGACG (SEQ ID NO: 28)
B3:AAAATTGGGGGGGGACTC (SEQ ID NO: 29)
LF: AGAGAAGTGAGAAGGAAATGAC (SEQ ID NO: 5)
LB:ATTACAAGGTTTCGGCCGATTT (SEQ ID NO: 30)

In accordance with the descriptions of Examples 1(c) and (d), various types of yeast strains were detected by the LAMP method using the aforementioned primer sets. The results are as shown in Figures 7 and 8.

In the case of using the primer set according to the present invention (DA1LF1LB1), a non-specific reaction was not observed although 60 minutes had passed after the initiation of the reaction (Figure 7). However, in the case of using the control primer set (DA2LF1LB1), when 60 minutes had passed after the initiation of the reaction, it reacted with yeast strains other than the yeast strain as a detection target (Figure 8).

Thus, it was demonstrated that the primer set according to the present invention has specificity significantly higher than that of a primer set in which a target position has been slightly changed, and that the primer set according to the present invention is able to accurately detect a target yeast strain.

## Claims

1. A LAMP primer set for use in the detection of Dekkera anomala, which comprises the following polynucleotides:
a polynucleotide (FIP) having the base sequence of SEQ ID NO: 1, or a polynucleotide consisting of at least 10 bases, which hybridizes with a polynucleotide having a sequence complementary to the base sequence (polynucleotide 1);
a polynucleotide (F3) having the base sequence of SEQ ID NO: 2, or a polynucleotide consisting of at least 10 bases, which hybridizes with a polynucleotide having a sequence complementary to the base sequence (polynucleotide 2);
a polynucleotide (BIP) having the base sequence of SEQ ID NO: 3, or a polynucleotide consisting of at least 10 bases, which hybridizes with a polynucleotide having a sequence complementary to the base sequence (polynucleotide 3); and
a polynucleotide (B3) having the base sequence of SEQ ID NO: 4, or a polynucleotide consisting of at least 10 bases, which hybridizes with a polynucleotide having a sequence complementary to the base sequence (polynucleotide 4).

2. The primer set according to claim 1, which further comprises any one or both of the following polynucleotides:
a polynucleotide (LF) having the base sequence of SEQ ID NO: 5, or a polynucleotide consisting of at least 10 bases, which hybridizes with a polynucleotide having a sequence complementary to the base sequence (polynucleotide 5); and
a polynucleotide (LB) having the base sequence of SEQ ID NO: 6, or a polynucleotide consisting of at least 10 bases, which hybridizes with a polynucleotide having a sequence complementary to the base sequence (polynucleotide 6).

3. A LAMP primer set for use in the detection of Dekkera bruxellensis, which comprises the following polynucleotides:
a polynucleotide (FIP) having the base sequence of SEQ ID NO: 7, or a polynucleotide consisting of at least 10 bases, which hybridizes with a polynucleotide having a sequence complementary to the base sequence (polynucleotide 7);
a polynucleotide (F3) having the base sequence of SEQ ID NO: 8, or a polynucleotide consisting of at least 10 bases, which hybridizes with a polynucleotide having a sequence complementary to the base sequence (polynucleotide 8);
a polynucleotide (BIP) having the base sequence of SEQ ID NO: 9, or a polynucleotide consisting of at least 10 bases, which hybridizes with a polynucleotide having a sequence complementary to the base sequence (polynucleotide 9); and
a polynucleotide (B3) having the base sequence of SEQ ID NO: 10, or a polynucleotide consisting of at least 10 bases, which hybridizes with a polynucleotide having a sequence complementary to the base sequence (polynucleotide 10).

4. The primer set according to claim 3, which further comprises a polynucleotide (LF) having the base sequence of SEQ ID NO: 11, or a polynucleotide consisting of at least 10 bases, which hybridizes with a polynucleotide having a sequence complementary to the base sequence (polynucleotide 11).

5. A LAMP primer set for use in the detection of Dekkera custersiana, which comprises the following polynucleotides:
a polynucleotide (FIP) having the base sequence of SEQ ID NO: 12, or a polynucleotide consisting of at least 10 bases, which hybridizes with a polynucleotide having a sequence complementary to the base sequence (polynucleotide 12);
a polynucleotide (F3) having the base sequence of SEQ ID NO: 13, or a polynucleotide consisting of at least 10 bases, which hybridizes with a polynucleotide having a sequence complementary to the base sequence (polynucleotide 13);
a polynucleotide (BIP) having the base sequence of SEQ ID NO: 14, or a polynucleotide consisting of at least 10 bases, which hybridizes with a polynucleotide having a sequence complementary to the base sequence (polynucleotide 14); and
a polynucleotide (B3) having the base sequence of SEQ ID NO: 15, or a polynucleotide consisting of at least 10 bases, which hybridizes with a polynucleotide having a sequence complementary to the base sequence (polynucleotide 15).

6. The primer set according to claim 5, which further comprises a polynucleotide (LB) having the base sequence of SEQ ID NO: 16, or a polynucleotide consisting of at least 10 bases, which hybridizes with a polynucleotide having a sequence complementary to the base sequence (polynucleotide 16).

7. A LAMP primer set for use in the detection of Brettanomyces naardenensis, which comprises the following polynucleotides:
a polynucleotide (FIP) having the base sequence of SEQ ID NO: 17, or a polynucleotide consisting of at least 10 bases, which hybridizes with a polynucleotide having a sequence complementary to the base sequence (polynucleotide 17);
a polynucleotide (F3) having the base sequence of SEQ ID NO: 18, or a polynucleotide consisting of at least 10 bases, which hybridizes with a polynucleotide having a sequence complementary to the base sequence (polynucleotide 18);
a polynucleotide (BIP) having the base sequence of SEQ ID NO: 19, or a polynucleotide consisting of at least 10 bases, which hybridizes with a polynucleotide having a sequence complementary to the base sequence (polynucleotide 19); and
a polynucleotide (B3) having the base sequence of SEQ ID NO: 20, or a polynucleotide consisting of at least 10 bases, which hybridizes with a polynucleotide having a sequence complementary to the base sequence (polynucleotide 20).

8. The primer set according to claim 7, which further comprises a polynucleotide (LF) having the base sequence of SEQ ID NO: 21, or a polynucleotide consisting of at least 10 bases, which hybridizes with a polynucleotide having a sequence complementary to the base sequence (polynucleotide 21).

9. The primer set according to any one of claims 1 to 8, wherein the polynucleotide consisting of at least 10 bases hybridizing with a polynucleotide having a sequence complementary to any one of the base sequences of SEQ ID NOS: 1 to 21 comprises at least 10 contiguous nucleotides of the corresponding base sequence.

10. The primer set according to any one of claims 1 to 8, wherein the polynucleotide consisting of at least 10 bases hybridizing with a polynucleotide having a sequence complementary to any one of the base sequences of SEQ ID NOS: 1 to 21 has at least 90% identity with the corresponding base sequence.

11. The primer set according to any one of claims 1 to 8, wherein the polynucleotide consisting of at least 10 bases hybridizing with a polynucleotide having a sequence complementary to any one of the base sequences of SEQ ID NOS: 1 to 21 is a polynucleotide which consists of a modified base sequence in which one or several mutations are introduced with respect to the corresponding base sequence, and hybridizes with the polynucleotide having the sequence complementary to the corresponding base sequence.

12. A method for detecting Dekkera anomala, which comprises performing a nucleic acid amplification reaction by a LAMP method using the primer set according to claim 1 or 2.

13. A method for detecting Dekkera bruxellensis, which comprises performing a nucleic acid amplification reaction by a LAMP method using the primer set according to claim 3 or 4.

14. A method for detecting Dekkera custersiana, which comprises performing a nucleic acid amplification reaction by a LAMP method using the primer set according to claim 5 or 6.

15. A method for detecting Brettanomyces naardenensis, which comprises performing a nucleic acid amplification reaction by a LAMP method using the primer set according to claim 7 or 8.

16. A kit for detecting a yeast of genus Dekkera and/or a yeast of genus Brettanomyces, which comprises a primer set selected from the group consisting of the primer set according to claim 1 or 2, the primer set according to claim 3 or 4, the primer set according to claim 5 or 6, the primer set according to claim 7 or 8, and a combination of these primer sets.

17. A kit for use in the quality control of alcoholic beverages and/or soft drinks, which comprises a primer set selected from the group consisting of the primer set according to claim 1 or 2, the primer set according to claim 3 or 4, the primer set according to claim 5 or 6, the primer set according to claim 7 or 8, and a combination of these primer sets.


SEQUENCE LISTING

<110>  Kirin Beer Kabushiki Kaisha

<120>  Primer sets for detecting Dekkera genus yeasts and Brettanomyces g
enus yeasts

<130>  165808PX

<150>  JP 136810/2006
<151>  2006-05-16

<160>  30

<170>  PatentIn version 3.1

<210>  1
<211>  42
<212>  DNA
<213>  Artificial

<220>
<223>  primer set (DA1LF1LB1)

<400>  1
accgcgtatt ctcataacca ctccgggcat gcctgtttga gc                    42


<210>  2
<211>  20
<212>  DNA
<213>  Artificial

<220>
<223>  primer set (DA1LF1LB1)

<400>  2
agttcttgaa cgcacattgc                                             20


<210>  3
<211>  51
<212>  DNA
<213>  Artificial

<220>
<223>  primer set (DA1LF1LB1)

<400> 3
actgggagga gtaataatta caaggtttcg gggactcagt aggagttgct a 51

<210> 4
<211> 21
<212> DNA
<213> Artificial

<220>
<223> primer set (DA1LF1LB1)

<400> 4
tcctcctacc tgatttgagg t 21

<210> 5
<211> 22
<212> DNA
<213> Artificial

<220>
<223> primer set (DA1LF1LB1)

<400> 5
agagaagtga gaaggaaatg ac 22

<210> 6
<211> 19
<212> DNA
<213> Artificial

<220>
<223> primer set (DA1LF1LB1)

<400> 6
gccgatttat tgcgtcttc 19

<210> 7
<211> 45
<212> DNA
<213> Artificial

<220>
<223> primer set (DB1LF1)

<400> 7
accctcgtgt aatctcataa ccactaagga gggcatgcct gtttg 45

<210> 8
<211> 16
<212> DNA
<213> Artificial

<220>
<223> primer set (DB1LF1)

<400> 8
acattgcgcc ctctgg 16

<210> 9
<211> 51
<212> DNA
<213> Artificial

<220>
<223> primer set (DB1LF1)

<400> 9
gatttaaggt ttcggccgtt cattattttc tcctacctga tttgaggtca a 51

<210> 10
<211> 19
<212> DNA
<213> Artificial

<220>
<223> primer set (DB1LF1)

<400> 10
tgcttaagtt cagcgggtc 19

<210> 11
<211> 19
<212> DNA
<213> Artificial

<220>
<223> primer set (DB1LF1)

<400>  11
agtgagaagg aaatgacgc                                                    19


<210>  12
<211>  39
<212>  DNA
<213>  Artificial

<220>
<223>  primer set (DC2LB1)

<400>  12
cggtgccgtc atcacaacta cgggcatgcc tgtttgagc                              39


<210>  13
<211>  20
<212>  DNA
<213>  Artificial

<220>
<223>  primer set (DC2LB1)

<400>  13
agttcttgaa cgcacattgc                                                   20


<210>  14
<211>  46
<212>  DNA
<213>  Artificial

<220>
<223>  primer set (DC2LB1)

<400>  14
gcggtccgtc gaagagaatg acgtagataa aagtcaacga ctggtc                      46


<210>  15
<211>  21
<212>  DNA
<213>  Artificial

<220>
<223>  primer set (DC2LB1)

<400>  15
tcctcctacc tgatttgagg t                                                21

<210>  16
<211>  20
<212>  DNA
<213>  Artificial

<220>
<223>  primer set (DC2LB1)

<400>  16
agttaacgga gtaaggtttc                                                  20

<210>  17
<211>  46
<212>  DNA
<213>  Artificial

<220>
<223>  primer set (BN1LF1)

<400>  17
gagaggtatc ttcaaaacca cttgcgattc tggggggtat tcctgt                     46

<210>  18
<211>  20
<212>  DNA
<213>  Artificial

<220>
<223>  primer set (BN1LF1)

<400>  18
agttcttgaa cgcacattgc                                                  20

<210>  19
<211>  52
<212>  DNA
<213>  Artificial

<220>
<223>  primer set (BN1LF1)

<400>  19
ggaattctga atttagttgg cttcttgttt gctttcaacg aatggtcgaa ac          52

<210>  20
<211>  22
<212>  DNA
<213>  Artificial

<220>
<223>  primer set (BN1LF1)

<400>  20
aaacgtcaag gtcattgact ac                                            22

<210>  21
<211>  21
<212>  DNA
<213>  Artificial

<220>
<223>  primer set (BN1LF1)

<400>  21
tgagaaggaa atgacgctca a                                             21

<210>  22
<211>  28
<212>  DNA
<213>  Artificial

<220>
<223>  sense primer

<400>  22
gataaaaata cattaaattt atttagtt                                      28

<210>  23
<211>  21
<212>  DNA
<213>  Artificial

<220>
<223>  antisense primer

<400>  23
gaacggccga aaccttaaat c                                                21

<210>  24
<211>  20
<212>  DNA
<213>  Artificial

<220>
<223>  primer set (DC1)

<400>  24
aacaaggttt ccgtaggtga                                                  20

<210>  25
<211>  25
<212>  DNA
<213>  Artificial

<220>
<223>  primer set (DC1)

<400>  25
aattcacatt agttatcgca attcg                                            25

<210>  26
<211>  46
<212>  DNA
<213>  Artificial

<220>
<223>  primer set (DC1)

<400>  26
catgtgttat aatgcttgca ctcggctgcg gaaggatcat taacga                     46

<210>  27
<211>  52
<212>  DNA
<213>  Artificial

<220>
<223>  primer set (DC1)

<400>  27
gcatacaaac acaacaaacc aaaatttatc aacttcatcg acgcgagaac ca          52

<210>  28
<211>  41
<212>  DNA
<213>  Artificial

<220>
<223>  primer set (DA2LF1LB1)

<400>  28
ccggtgggga gtatactggg agtaggagtt gctagaagac g          41

<210>  29
<211>  18
<212>  DNA
<213>  Artificial

<220>
<223>  primer set (DA2LF1LB1)

<400>  29
aaaattgggg ggggactc          18

<210>  30
<211>  22
<212>  DNA
<213>  Artificial

<220>
<223>  primer set (DA2LF1LB1)

<400>  30
attacaaggt ttcggccgat tt          22